Europäisches Patentamt

European Patent Office

Office européen des brevets

(19)

(11) Publication number:

# 0 220 063
## A2

(12)

# EUROPEAN PATENT APPLICATION

(21) Application number: 86308027.1

(22) Date of filing: 16.10.86

(51) Int. Cl.⁴: **C 12 P 21/00**
C 07 K 3/20, G 01 N 33/577

(30) Priority: 17.10.85 JP 233004/85

(43) Date of publication of application:
29.04.87 Bulletin 87/18

(84) Designated Contracting States:
BE CH DE ES FR GB IT LI NL SE

(71) Applicant: Dainippon Pharmaceutical Co., Ltd.
25, Doshomachi 3-chome Higashi-ku
Osaka-shi, Osaka 541 (JP)

(72) Inventor: Yamada, Masaaki
13, Murasakino-Nishiren-Daino-Cho Kita-ku
Kyoto-shi Kyoto-fu (JP)

Kawata, Shigeo
15-12, Wakabadai 2-chome Kita-ku
Kobe-shi Hyogo-ken (JP)

Sunahara, Noriyuki
86-2, Kawashimaarisugawa-cho Nishikyo-ku
Kyoto-shi Kyoto-fu (JP)

Furuta, Ryuji
24-8, Fujimidai
Otsu-shi Shiga-ken (JP)

Yamayoshi, Michiko
8-14, Nishi-Midorigaoka 3-chome
Toyonaka-shi Osaka-fu (JP)

(74) Representative: Harrison, David Christopher et al
MEWBURN ELLIS & CO 2/3 Cursitor Street
London EC4A 1BQ (GB)

(54) Anti-human interleukin 1 antibody, method for the production thereof and use of the same.

(57) An antibody, especially a monoclonal antibody, against a human interleukin I polypeptide having a particular amino acid sequence may be produced by forming a hybridoma cell between an antibody-producing cell of an animal immunized with the polypeptide and a myeloma cell, cloning the hybridoma cell and producing the anti-polypeptide antibody with a selected clone capable of such production.

The antibody can be used for the purification of the polypeptide and for the quantitative determination of human interleukin I by assays such as EIA and RIA.

**Description**

ANTI-HUMAN INTERLEUKIN 1 ANTIBODY, METHOD FOR THE PRODUCTION THEREOF AND USE OF THE SAME

This invention relates to an antibody against human interleukin 1 polypeptide (the human interleukin 1 is hereinafter referred to as "IL-1"), and a method for the production thereof. More particularly, it relates to an antibody against a polypeptide having an amino acid sequence of the following formula (I) and its monoclonal antibody, and a method for the production thereof.

Ser Ser Pro Phe Ser Phe Leu Ser Asn Val Lys Tyr Asn Phe Met
Arg Ile Ile Lys Tyr Glu Phe Ile Leu Asn Asp Ala Leu Asn Gln
Ser Ile Ile Arg Ala Asn Asp Gln Tyr Leu Thr Ala Ala Ala Leu
His Asn Leu Asp Glu Ala Val Lys Phe Asp Met Gly Ala Tyr Lys
Ser Ser Lys Asp Asp Ala Lys Ile Thr Val Ile Leu Arg Ile Ser
Lys Thr Gln Leu Tyr Val Thr Ala Gln Asp Glu Asp Gln Pro Val
Leu Leu Lys Glu Met Pro Glu Ile Pro Lys Thr Ile Thr Gly Ser
Glu Thr Asn Leu Leu Phe Phe Trp Glu Thr His Gly Thr Lys Asn
Tyr Phe Thr Ser Val Ala His Pro Asn Leu Phe Ile Ala Thr Lys
Gln Asp Tyr Trp Val Cys Leu Ala Gly Gly Pro Pro Ser Ile Thr
Asp Phe Gln Ile Leu Glu Asn Gln Ala [I]

IL-1 is a component in human body which has close relation with immune response in human body and the content thereof is variable with various diseases, such as immunodeficiency, inflammation, rheumatoid, and the like. Accordingly, when the content of IL-1 in body liquids can be rapidly determined with high sensitivity, it is very useful for diagnosis of these diseases, and further for the determination of the therapeutic manner of the diseases and for prognosis thereof.

Brief Summary of the Invention

The present inventors have succeeded in the production of an antibody against IL-1 and have found that by using the antibody, a slight amount of IL-1 can be determined in high sensitivity and with high accuracy by means of RIA (radioimmunoassay), EIA (enzyme immunoassay) or other assays and further that the antibody is useful for the purification or neutralization of IL-1.

An object of the invention is to provide an antibody against IL-1 polypeptide. Another object of the invention is to provide a monoclonal antibody against a polypeptide having an amino acid sequence of the formula [I]. A further object of the invention is to provide a method for the production of the antibody as set forth above. Still further object of the invention is to provide a use of the antibody for the determination of IL-1 by RIA, EIA or other assays. These and other objects and advantages of the invention will be apparent to skilled persons by the following description.

Brief Description of Drawing

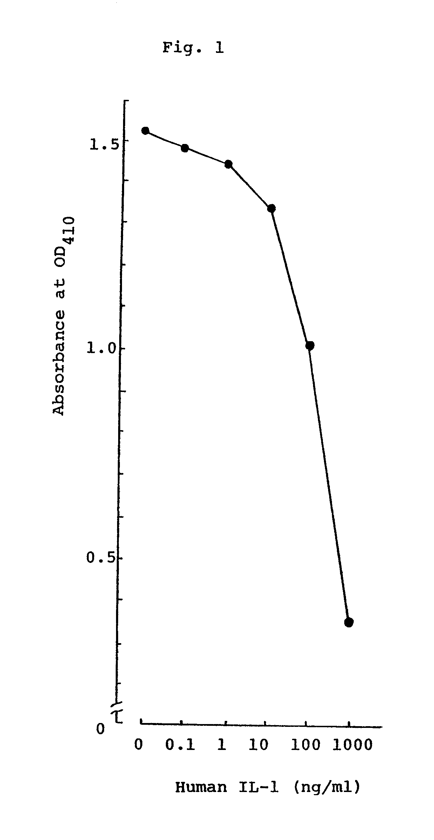

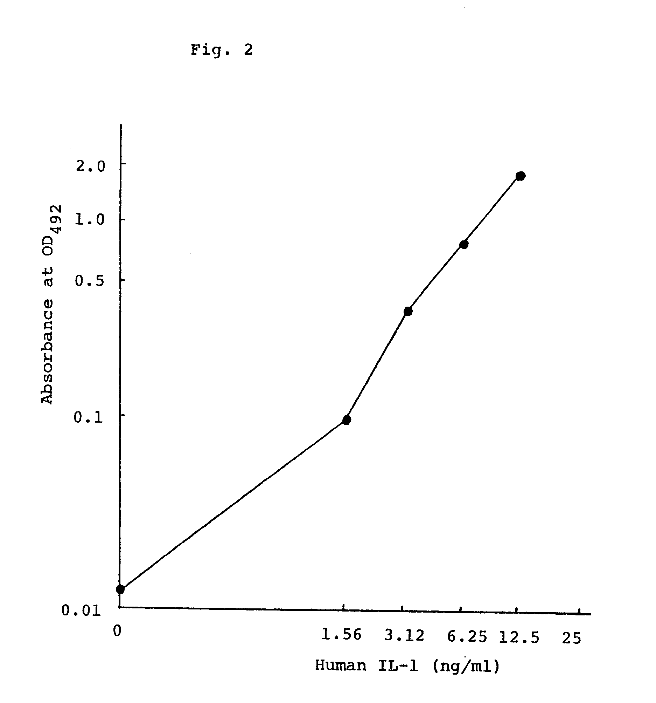

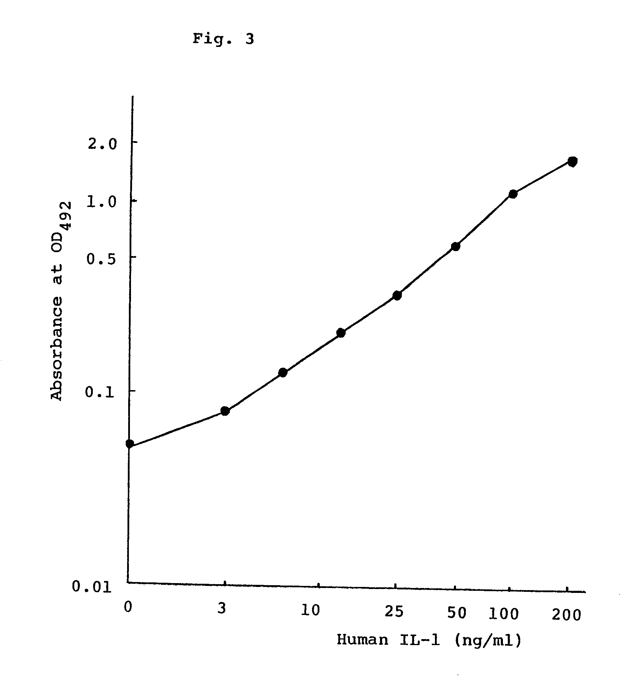

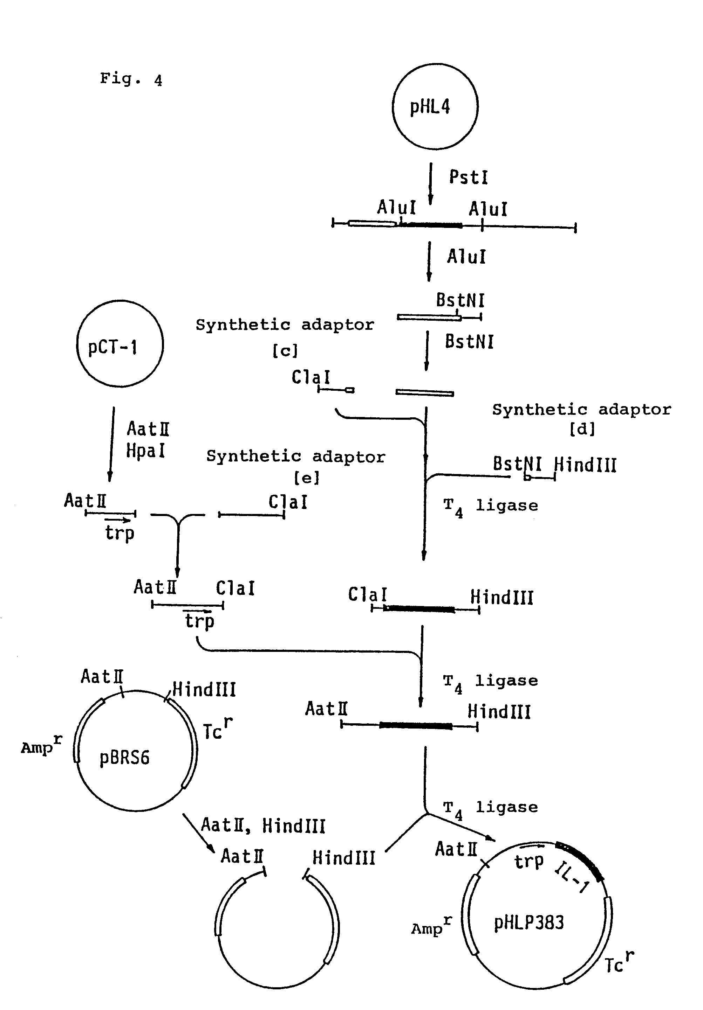

Fig. 1 shows a calibration curve in the determination of human IL-1 by EIA (competition method), Fig. 2 and Fig. 3 show a calibration curve in the determination of human IL-1 by EIA (sandwich method), and Fig. 4 shows the step for construction of the expression vector pHLP383, wherein the formulae [III], [IV] and [V] mean the oligodeoxyribonucleotide adaptors shown in Reference Example 1, respectively.

Detailed Description of the Invention

The antibody of this invention can be produced by immunizing an appropriate mammal (e.g. mouse, rat, etc.) with a polypeptide having an amino acid sequence of the formula [I] by a conventional method. Among the antibodies of the invention, monoclonal antibody is particularly useful and is prepared in the following manner.

A mammal (e.g. mouse, rat, etc.) is immunized with the polypeptide [I], and the spleen of the immunized animal is taken out at an appropriate time and the spleen cells are collected. The spleen cells are fused with myeloma cells in a conventional manner, for instance, by using a fusion promoter such as polyethylene glycol, to obtain hybridoma. The hybridoma is cultured and then the desired monoclonal antibody is isolated from the culture medium. The method is explained in more detail below.

The myeloma cells are preferably originated from the same animal as the animal used for the immunization and are not grown in the presence of aminopterin and do not secrete any antibody. Suitable examples of the myeloma cells are P3-X63-Ag8.653, SP2/0-Ag14, and the like.

The hybridoma cells obtained by the cell fusion are well washed with a medium [e.g. RPMI-1640 medium supplemented with 10 - 20 % fetal calf serum (FCS)], by which the fusion promoter is completely removed. The cells thus treated are cultured in the HAT medium containing hypoxanthine-aminopterin-thymidine (which is usually kept in a multi-tray having 96 wells) in 5 % $CO_2$-containing air at 37°C at a humidity of 90 to 100 %, by which the unfused myeloma cells do not survive and only the fused hybridoma cells are grown. After one or two weeks, the hybridoma which have produced the desired antibody are selected by determining whether the antibody is contained in the culture medium collected from each well.

The detection of the antibody is carried out by a known method, for instance, by reacting the culture medium with an enzyme- or isotope-labelled polypeptide [I], adding thereto an insolubilized antibody against immunoglobin of the animal which has been used for the preparation of the antibody, and then measuring the activity of the labelled substance, i.e. the enzymatic activity of the enzyme or radioactivity of the isotope (by so-called competition method).

By cloning the hybridoma cells which have produced the antibody by a known technique, for example, by a limiting dilution method, there can be obtained cloned cells. When the cloned cells are cultured in a medium, the desired monoclonal antibody is produced in the culture medium. Beside, when the cloned cells are transplanted intraperitoneally into a histocompatible animal or a thymectomized nude mouse, etc., followed by growing, there can be obtained the desired monoclonal antibody from the peritoneal liquid or blood of the animal.

The monoclonal antibody may be purified by conventional methods, for example, centrifugation, salting-out, dialysis, ion exchange chromatography, affinity chromatography, gel filtration, and the like.

The antibody against the polypeptide [I] of this invention is useful for the purification of the polypeptide [I], and this invention provides a method for the purification of the polypeptide [I].

The purification of the polypeptide [I] by using the antibody of this invention can be done as follows:

The anti-polypeptide [I] antibody is bound to an appropriate carrier (e.g. resin carrier) to prepare an insolubilized antibody. For example, the antibody is bound to Sepharose (manufactured by Pharmacia) activated with cyanogen bromide. By using the insolubilized antibody, polypeptide [I] containing materials such as body liquid, animal cells, microorganisms transformed with the expression vector producing the polypeptide [I] or culture thereof are subjected to affinity chromatography, by which the poly peptide [I] contained therein can be isolated and purified.

The starting human IL-1 polypeptide used in this invention is prepared by the method as described in Reference Example 1 hereinafter and is described in U.S. serial No. 812,796 and European Patent Application No. 85309453.0.

The antibody of this invention can also be used for the determination of polypeptide [I], and hence, this invention provides also an agent for the quantitative determination of polypeptide [I] by RIA, EIA or other assays, particularly by competition method or sandwich method.

The quantitative determination of IL-1 has hitherto been carried out by measuring the biological activity utilizing as an index an activity of LAF (lymphocyte activating factor) [cf. Gery et al, Cell. Immun., 64, 293 (1981)]. However, this method requires a very long time for the measurement (usually 3 to 4 days) and further is less in the accuracy of the data. By using the antibody of this invention, there can be measured the IL-1 rapidly and with high accuracy of data, and hence, this invention will be able to contribute greatly on the progress of the research of IL-1.

The agent for the quantitative determination of the polypeptide [I] of this invention comprises:

Component (A): an insolubilized antibody against polypeptide [I], and

Component (B): a labelled antibody, said antibody being an antibody against a polypeptide [I] which is different from the antibody in Component (A), or a labelled polypeptide [I].

Among the Component (B), the labelled antibody is used in the sandwich method, and the antibody has a recognition site different from that of the antibody used in Component (A). For instance, when the antibody for Component (A) is #1190 antibody, the antibody in Component (B) is #0964 as mentioned hereinafter. Besides, the labelled polypeptide [I] as Component (B) is used in the competition method.

The agent for the quantitative determination of polypeptide [I] of this invention may optionally be incorporated with conventional components in addition to Components (A) and (B), such as a solution containing a standard polypeptide [I] for preparing a calibration curve, reagents for measuring the activity of the labelled material (for instance, in case of enzyme-labelled antibody, a substrate, a liquid for dissolving the substrate, a liquid for the termination of the enzymatic reaction, etc.), a buffer, and the like.

The insolubilized antibody against the polypeptide [I] of Component (A) can be prepared by insolubilizing the antibody by a conventional method, for example, by binding the antibody to insoluble carriers, such as bacterial cell walls, natural insoluble polysaccharides, chemically treated dextran gel, agar gel, plastic beads, acrylamide gel, glass beads, fine metallic particles, synthetic rubber tube, and the like.

The labelling of the antibody or the polypeptide [I] can also be done by conventional methods with labelling agents, such as enzyme (e.g. peroxidase, etc.), radio-isotopes (e.g. $^{125}I$, $^{131}I$ etc.), fluorescent agents, spin compounds, and the like.

The procedure of the measurement of the polypeptide [I] by this invention is explained below.

[In case of a competition method]:

1. A test sample is reacted with Component (A): an insolublized antibody and Component (B): a labelled polypeptide [I], and thereby, the polypeptide [I] contained in the test sample and the labelled polypeptide [I] [Component (B)] react competitively with the insolubilized antibody [Component (A)].
2. The reaction mixture is centrifuged to separate the liquid phase and the solid phase.
3. The activity of the labelling agent in the liquid phase or the solid phase is measured.

[In case of a sandwich method]:

1. A test sample containing a polypeptide [I] is mixed with Component (A): an insolubilized antibody, whereby, the polypeptide [I] reacts with the insolubilized antibody.
2. The reaction mixture is washed to remove unreacted polypeptide [I].

3. Component (B): a labelled antibody against the polypeptide [I] is added to the washed reaction mixture and thereby the labelled antibody is reacted with the polypeptide [I] bound with the insolubilized antibody.

4. The reaction mixture is washed to remove unreacted labelled antibody.

5. The activity of the labelling agent in the [labelled antibody-polypeptide [I]-insolublized antibody] complex is measured.

In the above procedure, the steps 1 and 3 may be carried out simultaneously. That is, both of Component (A) and Component (B) may simultaneously be mixed with the test sample.

According to the above measurement, the concentration of the polypeptide [I] in the test sample can be calculated based on a calibration curve which is previously prepared by utilizing a standard polypeptide [I] solution having a prescribed concentration of polypeptide [I].

As mentioned above, in case of a sandwich method, the antibody in Component (B) should be an antibody against polypeptide [I] which is different from the antibody in Component (A). The antibody against polypeptide [I] includes various antibodies which are different in the binding sites in the polypeptide [I] (i.e. antigen). The kinds of the antibody can be determined by effect on the biological activity of human IL-1 (i.e. LAF activity) as is explained below.

Firstly, the LAF activity was determined as follows:

Human IL-1 was previously diluted with a medium suitable for tissue culture (e.g. RPM-1640 medium containing 5 % fetal calf serum). The diluted human IL-1 solution (50 µl) and an anti-human IL-1 antibody solution (or medium) (50 µ1) were added into a flat bottom microplate having 96 wells (manufactured by Flow Labs.), and to each well was added a suspension (each 100 µl) of C3H/He mouse thymus cells (1 x 10⁷ cell/ml) containing phytohemagglutinine-P (manufactured by Difco Labs., concentration: 12.5 µg/ml), and the mixture was cultivated in 5 % $CO_2$-containing air at 37°C at a humidity of 90 to 100 % for 2 days. Thereafter, ³H-thymidine (1 µCi) was added thereto, the mixture was further cultivated for 18 hours. After the culture, the ³H-tymidine incorporated in the cells was counted and thereby the LAF activity was calculated. From the data thus measured, the effect of anti-human IL-1 on LAF activity (i.e. inhibition of LAF activity) was evaluated. The results are shown in Table 1.

Table 1

| Kind of antibody | Inhibition of LAF activity[**] | |
|---|---|---|
| | Amount of human IL-1: 1.0 ng/ml | Amount of human IL-1: 0.1 ng/ml |
| (Monoclonal antibody) #0234 #0374 #0520 #0964 #1190 | 40.2 % 9.4 " 27.2 " 29.0 " 58.0 " | 51.3 % 0 " 30.9 " 48.1 " 83.3 " |
| (Polyclonal antibody[*]) 40-fold diluted 200-fold diluted | 90.4 % 72.2 " | 85.2 % 68.0 " |

[Note]: *) Anti-human IL-1 polyclonal antiserum as prepared in Example 2 hereinafter.
**) The inhibition was calculated by the following formula:

$$[(a - b)/a] \times 100 \ (\%)$$

a: ³H-thymidine incorporation amount in case of no antibody being added,

b: ³H-thymidine incorporation amount in case of an antibody being added.

As is clear from the above results, the inhibitory effect of the monoclonal antibody against the LAF activity of human IL-1 varies in each monoclonal antibody, and it was stronger in the order of

#1190 > #0234 > #0964 > #0520.

Particularly, the monoclonal antibody #1190 showed extremely higher inhibitory activity, and hence, it may be an antibody which can recognize the active site for the LAF activity of IL-1. On the other hand, the monoclonal antibody #0374 showed little effect on the LAF activity. All these monoclonal antibodies were of IgG$_1$ subclass.

For simplification of the description, the following abbreviations are used in the present specification.

A: adenine
C: cytosine
G: guanine
T: thymine
Ala: alanine
Arg: arginine
Asn: asparagine
Asp: aspartic acid
Cys: cysteine
Gln: glutamine
Glu: glutamic acid
Gly: glycine
His: histidine
Ile: isoleucine
Leu: leucine
Lys: lysine
Met: methionine
Phe: phenylalanine
Pro: proline
Ser: serine
Thr: threonine
Trp: tryptophan
Tyr: tyrosine
Val: valine
DNA: deoxyribonucleic acid
cDNA: complementary DNA
dATP: deoxyadenosine triphosphate
dCTP: deoxycytidine triphosphate
dGTP: deoxyguanosine triphosphate
dTTP: deoxythymidine triphosphate
EDTA: ethylenediaminetetraacetic acid
kbp: kilobase pairs
bp: base pairs

The following examples and reference example illustrate this invention more specifically. It should be understood however that the invention is in no way limited to these examples.

Example 1

Preparation of monoclonal antibody against human IL-1 polypeptide:

(1) Preparation of hybridomas:

Human IL-1 prepared in Reference Example 1-(2) (10 μg) was emulsified with Freund's complete adjuvant (manufactured by Difco Labs.), and the emulsion was interaperitoneally administered to BALB/c mouse. After about one month, an aqueous solution (0.2 ml) of human IL-1 (50 μg/ml) was intraperitoneally administered, and after 7 days, the same amount as above of human IL-1 was further administered likewise. Four days after the final immunization, the spleen was taken out and spleen cells were collected therefrom.

The spleen cells (1.8 x 10$^8$ cells) were mixed with mouse myeloma cells (P3X63-Ag8.653, 3 x 10$^7$ cells), and the mixture was centrifuged to remove the medium. The cells were pelletized, and thereto was added about 40 % polyethylene glycol (1ml) containing 15% dimethylsulfoxide which was kept at 37°C, and the mixture was slowly stirred. After one minute, RPMI-1640 medium (10 ml) warmed at 37°C was gradually added thereto.

The reaction mixture was centrifuged and the supernatant was removed. The resulting cell pellet was suspended in RPMI-1640 medium (36 ml) containing 0.1 mM hypoxanthine, 0.4 μM aminopterin, 16 μM thymidine and 15 % fetal calf serum. Each 0.1 ml of the suspension was added into each well of a 96 multi-well plate which were previously added with mouse thymocytes cells as the feeder layer, and then cultivated in 5 % CO$_2$-containing air at 37°C.

(2) Cloning:

Two weeks after the above culture, from the wells where the growth of cells was observed, the supernatant of the culture was collected, and the presence of an antibody against human IL-1 was checked in the following

5

manner.

That is, the culture supernatant (20 µl) was mixed with 10 µl of human IL-1 labelled with β-galactosidase (originated from Escherichia coli, maufactured by Boeringer Manheim) (cf. Example 3). The mixture was allowed to stand at 37°C for 30 minutes, and thereto was added a suspension of an insolubilized anti-mouse IgG rabbit antibody (cf. Example 3) (20µl), and the mixture was further allowed at 37°C for 15 minutes. A physiological saline solution (150 µl) was added thereto, and the mixture was centrifuged to separate the pellet. The pellet was suspended in Buffer A (0.1 % bovine serum albumin, 0.9 % sodium chloride, 0.04 M sodium phosphate; pH 7.0) (60 µl), and thereto was added an enzyme substrate for colorimetry (composition; 0.8 % 2-nitrophenyl-β-D-galactopyranoside, 1 mM MgCl₂, and 40 % ethylene glycol) (20 µl), and the mixture was allowed to stand at 37°C for 60 minutes, and then a reaction terminator (120 µl) was added to stop the enzymatic reaction. The reaction mixture was centrifuged, and the absorbance at $OD_{410}$ of the supernatant was measured.

As to the hybridoma on which high antibody value was observed, a cloning was carried out by a limiting dilution method, and thereby, there were obtained 10 cloned cells.

(3) Production of monoclonal antibody against human IL-1:

Each of the cloned cells as prepared in the above (2) was cultured on RPMI-1640 medium containing 15 % fetal calf serum in 75 cm² culture flask for 4 days. The supernatant of the culture was collected and centrifuged. The resulting supernatant showed high antibody value by measuring the antibody by the same detection method as mentioned in the above (2).

Example 2

Preparation of polyclonal antibody against human IL-1:

Under the same conditions as described in Example 1-(1), a mouse was immunized with human IL-1, and 7 days after the final immunization, whole blood was collected from the heart, and the serum was isolated therefrom to give anti-human IL-1 polyclonal antiserum.

Example 3

EIA (competition method) of human IL-1 with monoclonal antibody:

A solution of human Il-1 as prepared in Reference Example 1-(2) (0, 0.1, 1.0, 10, 100, and 1,000 ng/ml) (100 µl) was mixed with monoclonal antibody ( #0234) as prepared in Example 1-(3) (500-fold dilution, 100 µl), and the mixture was allowed to stand at 37°C for one hour, and thereto was added an enzyme-labelled human IL-1 solution (200 µl), and the mixture was further allowed to stand at 37°C. After one hour, a suspension of an insolubilized anti-mouse IgG rabbit antibody (200 µl) was added, and the mixture was allowed to stand at 37°C for one hour. A physiological saline solution (4 ml) was added thereto, and the mixture was centrifuged. The supernatant was removed by suction, and to the residue were added Buffer A (500 µl) and a solution (100 µl) of 0.8 % 2-nitrophenyl-β-galactopyranoside, 1 mM MgCl₂ and 40 % ethylene glycol, and the mixture was allowed to stand at 37°C for 30 minutes. To the mixture was added 0.1 M potassium phosphate solution (pH 11, 1.5 ml), and the mixture was centrifuged, and the resulting supernatant was subjected to colorimetry at 410 nm. Under this condition, the human Il-1 was accurately determined in the concentration range of human IL-1 of 0.1 to 1,000 ng/ml (cf. Fig. 1).

The enzyme-labelled human IL-1 was prepared as follows:

To a solution of human IL-1 in 50 mM sodium phosphate solution (175 µg/300 µl, pH 7.4) was added a solution of N-(m-maleimidobenzoyloxy)succinimide in dioxane (100 µg/50 µl), and the mixture was stirred at room temperature for 30 minutes, and thereto was added 0.5 M Tris-HCl (pH 7.5) (200 µl), and the mixture was further stirred for 10 minutes. To the reaction mixture was added 0.05 M Tris-HCl (pH 7.5) (1 ml), and the mixture was filtered with YM-5 filter membrane (manufactured by Amicone Co.), and the residue was washed twice with the same buffer as above (2 ml). The residue was dissolved in 0.05 M Tris-HCl (pH 7.0) (0.4 ml), and thereto was added an ammonium sulfate-saturated solution of E. coli β-D-galactosidase (2 mg/0.4 ml), and the mixture was stirred at room temperature for 3 hours. The solution was applied to a column of Sepharose 6B (manufactured by Pharmacia) and well washed with Buffer A, and thereby the fractions containing anti-human IL-1 antibody bound with β-D-galactosidase and having β-D-galactosidase activity were collected and diluted with Buffer A to give the desired enzyme-labelled human IL-1.

The insolubilized anti-mouse IgG antibody was prepared as follows:

To an aqueous suspension of cell walls of Lactobacillus plantarum (20 mg/2 ml) was added anti-mouse IgG rabbit antibody (manufactured by Miles Lab., 100 µl), and thereto were added with stirring 1M acetic acid-HCl solution (pH 4, 30 µl), 5 % aqueous solution of a water-soluble carbodiimide (60 µl) and 25 % aqueous glutalaldehyde solution (10 µl) in this order, and the mixture was stirred at room temperature for one hour. The reaction mixture was centrifuged (3,000 rpm, 10 minutes) to remove the supernatant, and to the residue was added Buffer A (5 ml), and the mixture was subjected to ultrasonic treatment (20 KHz, 5 seconds) twice, and the resultant was again centrifuged. The residue was diluted to 10 fold with the above bacterial cell wall suspension (2.5 mg/ml) to give the desired insoluble anti-mouse IgG rabbit antibody.

Example 4

EIA (sandwich method) of human IL-1 by monoclonal antibody:

(1) Purification of antibody:

Ascitic fluid previously was collected from BALB/c mice which were intraperitonerally injected with pristane (manufactured by Aldrich Co.) and were intraperitoneally transplanted with an anti-IL-1-producing hybridoma #1190. To the fluid (2 ml) was added an equivolume of 0.2 M phosphate buffer (pH 7.0), and thereto was added dropwise with stirring an aqueous saturated ammonium sulfate solution (4 ml) at 0°C, by which crude IgG faction was precipitated. The precipitate was dissolved in 0.2 M phosphate buffer (pH 7.0), and the solution was dialyzed against 0.04 M phosphate buffer (pH 7.0) containing 0.9 % NaCl and then purified with MAPS kit (manufactured by Bio-Rad Co.) using a protein A-bound affinity gel.

(2) Production of a microtiter plate coated with anti-human IL-1 monoclonal antibody #1190:

To a polystyrene microtiter plate (for EIA, manufactured by Flow Co.) was added each 100 μl/well of a diluted solution of the antibody as prepared in the above (1) [which was diluted so as to be 15 μg/ml with 0.15 M NaCl - 0.02 M phosphate buffer (referred to "PBS") (pH 7.0), and they was allowed to stand at 4°C overnight. The inner solution was removed, and the residue was washed with purified water three times, and thereto was added 300 μl/well of 1 % bovine serum albumin (BSA)-containing 0.02 M phosphate buffer (pH 7.0), and they were allowed to stand at 4°C overnight, by which the reactive site of the unreacted protein on the plate was saturated with BSA. After washing with purified water, 0.1 % NaN₃-0.1 % BSA-0.04 % phosphate buffer was added to each well, and then the plate was kept at 4°C.

(3) Preparation of enzyme-labelled antibody stock:

It was prepared in the following manner by using peroxidase as the enzyme by the method of Nakane et al [cf. J. Histochem. Cytochem., 82, 1084 (1974)].

Peroxidase I-c (manufactured by Toyoboseki K.K.) (4 mg) was dissolved in water (1 ml), and thereto was added 0.1 M NaIO₄ (0.2 ml), and the mixture was incubated at room temperature for 20 minutes. After the reaction, the reaction mixture was dialyzed against 1 mM acetate buffer (pH 4.5) at 4°C overnight. The dialyzed solution was adjusted to pH 9.5 with 0.2 M sodium carbonate and thereto was added anti-IL-1 monoclonal antibody #0964 (5 mg), said antibody being purified in the same manner as described in the above (1). The mixture was incubated at room temperature for 2 hours. To the mixture was added NaBH₄ solution (4 mg/ml, 0.1 ml), and the mixture was allowed to stand at 4°C for 2 hours and then subjected to gel filtration using a column of Sephacryl® S200 (manufactured by Pharmacia) in 0.02 M phosphate buffer (pH 7.0). The fractions containing an enzyme-labelled antibody (absorbance at OD₂₈₀ and OD₄₅₀) were collected and was kept at -20°C lyophilized as the enzyme-labelled antibody stock.

(4) Measurement of IL-1:

From the microtiter plate coated with anti-IL-1 monoclonal antibody #1190 as prepared in the above (2), the inner solution was removed, and then the plate was washed twice with purified water and then flapped. To the plate was added an IL-1 standard solution or a test sample (50 μl) and thereto was added 0.1 % BSA-0.02 M PBS (50 μl), and the mixture was incubated at 30°C for one hour. After removing the reaction solution, the plate was washed with 250 μl/well of 0.1 % Tween 20-0.02 M PBS (pH 7.0) three times. To the plate was added an enzyme-labelled antibody (100 μl) which was prepared by diluting in 400-fold the enzyme-labelled antibody stock prepared in (3) with 1 % BSA-0.02 M PBS (pH 7.0), and the mixture was reacted at 30°C for one hour. After removing unbound enzyme-labelled antibody, the wells of the plate were washed with 250 μl/well of 0.1 % Tween 20-0.02 M PBS (pH 7.0) three times. A previously prepared 0.25 % o-phenylenediamine solution (100 μl) [which was prepared by dissolving in 0.1 M sodium phosphate-citric acid buffer (pH 5.0) containing 0.15 % H₂O₂] was added to each well, and incubated at 30°C for 20 minutes. The enzymatic reaction was stopped by adding 1N H₂SO₄ (50 μl). The absorbance at OD₄₉₂ was measured with Titertek Multiskan® MC (manufactured by Flow Co.) using as a control 0.25 % o-phenylenediamine solution, and therefrom, a calibration curve was prepared (cf. Fig. 2). In comparison of the absorbance of the test sample with the calibration curve, the concentration of IL-1 in the test sample was calculated.

Example 5

EIA (sandwich method) of human IL-1 by using monoclonal antibody:

In the same manner as described in Example 4 except for using the insoluble monoclonal antibody #0374 and 100-fold diluted enzyme-labelled antibody stock, the quantitative determination of IL-1 was carried out. The calibration curve thus obtained is shown in Fig. 3.

## Example 6

Difference of antigen-determining group of anti-IL-1 monoclonal antibody:

Five kinds of anti-IL-1 monoclonal antibodies as shown in Table 2 were coated to a polystyrene microtiter plate, and thereto was bound IL-1 by antigen-antibody reaction. Thereto was reacted each peroxidase-labelled monoclonal antibody, and by the reactivity, the epitope of IL-1 recognized by monoclonal antibodies was determined.

### Table 2

| Coated antibody | Labelled antibody | | | | |
|---|---|---|---|---|---|
| | #0234 | #0374 | #0520 | #0964 | #1190 |
| #0234 | − | + | − | + | − |
| #0374 | + | − | + | + | + |
| #0520 | − | + | − | + | − |
| #0964 | + | + | + | − | + |
| #1190 | − | + | − | + | − |

From the above results, they were classified to the following groups.

Group 1: #0374
Group 2: #0964
Group 3: #0234, #0520, #1190

## Reference Example 1

Preparation of human IL-1:

(1) Construction of human IL-1 polypeptide producing transformant:

An expression plasmid (pHLP383) for producing human IL-1 polypeptide having an amino acid sequence of the formula [I] was constructed as illustrated in Fig. 4.

A recombinant plasmid pHL4 containing the cDNA encoding human IL-1 was prepared by the method of Furutani et al [Nucleic Acids Res., 13, 5869 (1985)]. The cloned cDNA was isolated by digestion with restriction endonuclease PSTI from the recombinant plasmid pHL4 and the cDNA was further digested with restriction endonuclease AluI to obtain a DNA fragment being about 533 bp in size corresponding to the DNA downstream from the base No. 351 in Table 3. Furthermore, the DNA fragment was digested with restriction endonuclease BstNI to isolate the DNA fragment corresponding to the base No. 351 to 808 in Table 3. The resulting DNA fragment was sequentially ligated by T4 ligase with chemically synthesized oligodeoxyribonucleotide adaptors represented by the following formulae.

5'-CGATTATGTCATCACCTTTTAG
3'-TAATACAGTAGTGGAAAATC [II]
and
5'-AGGCGTGATGA
3'-CCGCACTACTTCGA [III]

The DNA fragment having the initiation codon ATG to the 5' end of the DNA fragment encoding human IL-1 polypeptide [I] and the double stop codons TGATGA to the 3' end of it was constructed (hereinafter, referred to as "HIL-1 fragment").

About 380 bp DNA fragment containing the trp promoter region was cut out from a plasmid pCT-1 [Ikehara, M., et al., Proc. Natl. Acad. Sci. USA, 81, 5956 (1984)] by double digestion with restriction endonucleases HpaI and AatII and isolated. The DNA fragment was ligated by T4 ligase with a chemically synthesized oligodeoxyribonucleotide adaptor represented by the following formula. 5'-AACTAGTACGCAAGTTCACGTAAGGAGGTTAT
3'-TTGATCATGCGTTCAAGTGCATTCCTCCAATAGC [IV]

The resulting DNA fragment [IV] was ligated with the previously prepared HIL-1 fragment by T4 ligase (hereinafter referred to as the "promoter-HIL-1 fragment").

Separately, plasmid pBR322 was digested with restriction endonucleases Aval and Pvull, and the resulting larger DNA fragment (about 3.7 kbp in size) was isolated by 0.7 % agarose gel electrophoresis. After filling-in its cohesive ends to blunt-ends with E. coli DNA polymerase I (Klenow fragment) and dGTP, dATP, dCTP, dTTP, both ends were ligated by T4 ligase to construct a plasmid vector (designated pBRS6).

The plasmid pBRS6 vector was cleaved with restriction endonuclease AatII and HindIII into two fragments, and a larger DNA fragment (about 3.6 kbp) was isolated. Then, this fragment was ligated by T4 ligase with the promoter-HIL-1 fragment previously prepared in order to construct an expression plasmid for producing human IL-1 polypeptide [I] (this is designated as pHLP383).

The resulting expression plasmid pHLP383 was introduced into E. coli HB101 by the method described below.

That is, E. coli HB101 was innoculated in 5 ml of the L broth (composition: tryptone 10 g, yeast extract 5 g, NaCl 5 g, glucose 1 g per one liter, pH 7.2) and cultivated overnight at 37°C. One milliliter of the resulting culture was innoculated in 100 ml of L broth, and further cultivated at 37°C until the turbidity at 650 nm of the culture reached 0.6. After standing for 30 minutes in ice water, the cells were collected by centrifugation and suspended in 50 ml of 50 mM $CaCl_2$, followed by standing at 0°C for 60 minutes. The cells were then collected by centrifugtion and again suspended in 10 ml of 50 mM $CaCl_2$ containing 20 % glycerol.

To the suspension was added the expression vector pHLP383 as above, and it was incubated in ice water for 20 minutes, then at 42°C for 1 minute and further at room temperature for 10 minutes, and LB broth (composition: tryptone 10 g, yeast extract 5 g and NaCl 10 g per one liter, pH 7.5) was added. The mixture was shaken at 37°C for 60 minutes. An aliquot of the resulting cell suspension was seeded on LB agar plates containing 25 µg/ml of ampicillin, and cultivated overnight at 37°C. Then ampicillin-resistant colonies were selected to obtain transformants. One of the transformants was named HB101/pHLP383.

(2) Production of human IL-1 polypeptide and purification thereof:

The transformant HB101/pHLP383 obtained in the above (1) was cultivated overnight in the LB broth at 37°C. Ten milliliters of the culture were inoculated in 1 liter of the modified M9 medium (composition: 1.5 % $Na_2HPO_4 \cdot 12H_2O$, 0.3 % $KH_2PO_4$, 0.05 % NaCl, 0.1 % $NH_4Cl$, 2 mg/l vitamin $B_1$, 0.5 % casamino acid, 2 mM $MgSO_4$, 0.1 mM $CaCl_2$, 0.5 % glucose) and cultivated at 37°C for 1 hour. Then, 3-indoleacrylic acid was added to a final concentration of 20 µg/ml, and the cultivation was continued further for 24 hours. Then, the cells were collected by centrifugation. The cells were suspended in 100 ml of 50 mM Tris-HCl (pH 8.0) buffer containing 0.1 % lysozyme and 30 mM NaCl and allowed to stand at 0°C for 30 minutes. Then, the suspension was frozen in a dry ice/ethanol bath and thawed at 37°C. After this freezing-thawing procedure was repeated, 2 ml of a 10 % polyethyleneimine solution was added to the cell suspension and it was allowed to stand. The cell debris was removed by centrifugation to give a clarified extract.

The extract was mixed with an equal volume of saturated ammonium sulfate solution. After standing, a precipitate was collected by centrifugation. The precipitate was dissolved in about 100 ml of 20 mM Tris-HCl (pH 8.0) buffer, and dialyzed agaist the same buffer. The dialysate was applied onto the column of DEAE-Sepharose CL-6B which was previously equilibrated with the same buffer. The column was well washed with the same buffer, and eluted with a linear gradient of NaCl from 0 to 0.5 M. The fractions having IL-1 activity (LAF activity) were collected and pooled. Then, it was concentrated by ultrafiltration and subjected to gel filtration using a Sephacryl S-200 column. The fractions having IL-1 activity were collected and pooled. The purified preparation was obtained by repeating the above procedures of DEAE-Sepharose CL-6B column chromatography and Sephacryl S-200 gel filtration.

Table 3

```
1                                      30                                          60
ATG GCC AAA GTT CCA GAC ATG TTT GAA GAC CTG AAG AAC TGT TAC AGT GAA AAT GAA GAA
Met Ala Lys Val Pro Asp Met Phe Glu Asp Leu Lys Asn Cys Tyr Ser Glu Asn Glu Glu
                                      (10)                                        (20)


                                      90                                          120
GAC AGT TCC TCC ATT GAT CAT CTG TCT CTG AAT CAG AAA TCC TTC TAT CAT GTA AGC TAT
Asp Ser Ser Ser Ile Asp His Leu Ser Leu Asn Gln Lys Ser Phe Tyr His Val Ser Tyr
                                      (30)                                        (40)


                                      150                                         180
GGC CCA CTC CAT GAA GGC TGC ATG GAT CAA TCT GTG TCT CTG AGT ATC TCT GAA ACC TCT
Gly Pro Leu His Glu Gly Cys Met Asp Gln Ser Val Ser Leu Ser Ile Ser Glu Thr Ser
                        .             (50)                                        (60)


                                      210                                         240
AAA ACA TCC AAG CTT ACC TTC AAG GAG AGC ATG GTG GTA GTA GCA ACC AAC GGG AAG GTT
Lys Thr Ser Lys Leu Thr Phe Lys Glu Ser Met Val Val Val Ala Thr Asn Gly Lys Val
                                      (70)                                        (80)


                                      270                                         300
CTG AAG AAG AGA CGG TTG AGT TTA AGC CAA TCC ATC ACT GAT GAT GAC CTG GAG GCC ATC
Leu Lys Lys Arg Arg Leu Ser Leu Ser Gln Ser Ile Thr Asp Asp Asp Leu Glu Ala Ile
                                      (90)                                        (100)


                                      330                                         360
GCC AAT GAC TCA GAG GAA GAA ATC ATC AAG CCT AGG TCA TCA CCT TTT AGC TTC CTG AGC
Ala Asn Asp Ser Glu Glu Glu Ile Ile Lys Pro Arg Ser Ser Pro Phe Ser Phe Leu Ser
                                      (110)                                       (120)
```

                                                    --to be continued--

Table 3

```
                                          390                                         420
AAT GTG AAA TAC AAC TTT ATG AGG ATC ATC AAA TAC GAA TTC ATC CTG AAT GAC GCC CTC
Asn Val Lys Tyr Asn Phe Met Arg Ile Ile Lys Tyr Glu Phe Ile Leu Asn Asp Ala Leu
                                         (130)                                       (140)


                                          450                                         480
AAT CAA AGT ATA ATT CGA GCC AAT GAT CAG TAC CTC ACG GCT GCT GCA TTA CAT AAT CTG
Asn Gln Ser Ile Ile Arg Ala Asn Asp Gln Tyr Leu Thr Ala Ala Ala Leu His Asn Leu
                                         (150)                                       (160)


                                          510                                         540
GAT GAA GCA GTG AAA TTT GAC ATG GGT GCT TAT AAG TCA TCA AAG GAT GAT GCT AAA ATT
Asp Glu Ala Val Lys Phe Asp Met Gly Ala Tyr Lys Ser Ser Lys Asp Asp Ala Lys Ile
                                         (170)                                       (180)


                                          570                                         600
ACC GTG ATT CTA AGA ATC TCA AAA ACT CAA TTG TAT GTG ACT GCC CAA GAT GAA GAC CAA
Thr Val Ile Leu Arg Ile Ser Lys Thr Gln Leu Tyr Val Thr Ala Gln Asp Glu Asp Gln
                                         (190)                                       (200)


                                          630                                         660
CCA GTG CTG CTG AAG GAG ATG CCT GAG ATA CCC AAA ACC ATC ACA GGT AGT GAG ACC AAC
Pro Val Leu Leu Lys Glu Met Pro Glu Ile Pro Lys Thr Ile Thr Gly Ser Glu Thr Asn
                                         (210)                                       (220)


                                          690                                         720
CTC CTC TTC TTC TGG GAA ACT CAC GGC ACT AAG AAC TAT TTC ACA TCA GTT GCC CAT CCA
Leu Leu Phe Phe Trp Glu Thr His Gly Thr Lys Asn Tyr Phe Thr Ser Val Ala His Pro
                                         (230)                                       (240)
```

--to be continued--

## Table 3

```
                                              750                                            780
AAC TTG TTT ATT GCC ACA AAG CAA GAC TAC TGG GTG TGC TTG GCA GGG GGG CCA CCC TCT
Asn Leu Phe Ile Ala Thr Lys Gln Asp Tyr Trp Val Cys Leu Ala Gly Gly Pro Pro Ser
                                             (250)                                          (260)

                                              810                                            840
ATC ACT GAC TTT CAG ATA CTG GAA AAC CAG GCG TAG GTC TGG AGT CTC ACT TGT CTC ACT
Ile Thr Asp Phe Gln Ile Leu Glu Asn Gln Ala ***
                                             (270)
```

[Note]: 1) *** means the stop codon.
         2) The parenthesized number means the number of amino acids.

## Claims

1. An antibody against a polypeptide having an amino acid sequence of the following formula [I]:

Ser Ser Pro Phe Ser Phe Leu Ser Asn Val Lys Tyr Asn Phe Met
Arg Ile Ile Lys Tyr Glu Phe Ile Leu Asn Asp Ala Leu Asn Gln
Ser Ile Ile Arg Ala Asn Asp Gln Tyr Leu Thr Ala Ala Ala Leu
His Asn Leu Asp Glu Ala Val Lys Phe Asp Met Gly Ala Tyr Lys
Ser Ser Lys Asp Asp Ala Lys Ile Thr Val Ile Leu Arg Ile Ser
Lys Thr Gln Leu Tyr Val Thr Ala Gln Asp Glu Asp Gln Pro Val
Leu Leu Lys Glu Met Pro Glu Ile Pro Lys Thr Ile Thr Gly Ser
Glu Thr Asn Leu Leu Phe Phe Trp Glu Thr His Gly Thr Lys Asn
Tyr Phe Thr Ser Val Ala His Pro Asn Leu Phe Ile Ala Thr Lys
Gln Asp Tyr Trp Val Cys Leu Ala Gly Gly Pro Pro Ser Ile Thr
Asp Phe Gln Ile Leu Glu Asn Gln Ala [I]

2. The antibody according to claim 1 which is a monoclonal antibody.

3. A method for producing an antibody against a polypeptide [I], which comprises forming hybridoma cell between an antibody-producing cell of an animal immunized with the polypeptide having an amino acid sequence of the formula [I] as set forth in claim 1 with myeloma cell, cloning the hybridoma cell, selecting a clone being capable of producing the anti-polypeptide [I] antibody, and producing the anti-polypeptide [I] monoclonal antibody with the clone.

4. A method for the purification of a polypeptide [I], which comprises subjecting a crude polypeptide having an amino acid sequence of the formula [I] as set forth in claim 1 to an affinity chromatography with a resin carrier bound with an antibody against said polypeptide.

5. A kit for the quantitative determination of a polypeptide having an amino acid sequence of the formula [I] as set forth in claim 1, which comprises

Component (A): an insolubilized antibody against a polypeptide [I], and

Component (B): a labelled antibody which is different from the antibody in Component (A), or a labelled polypeptide [I].

0220063

Fig. 1

Absorbance at $OD_{410}$

Human IL-1 (ng/ml)

Fig. 2

0220063

Fig. 3

Fig. 4

0220063